(19) Europäisches Patentamt
European Patent Office
Office européen des brevets

(11) **EP 3 662 305 B1**

(12) **EUROPEAN PATENT SPECIFICATION**

(45) Date of publication and mention
of the grant of the patent:
**19.07.2023 Bulletin 2023/29**

(21) Application number: **18755920.8**

(22) Date of filing: **30.07.2018**

(51) International Patent Classification (IPC):
**G01T 1/14** *(2006.01)*      **G01T 1/29** *(2006.01)*

(52) Cooperative Patent Classification (CPC):
**G01T 1/14; G01T 1/29**

(86) International application number:
**PCT/IB2018/055666**

(87) International publication number:
**WO 2019/025933 (07.02.2019 Gazette 2019/06)**

(54) **METHOD FOR MEASURING RADIOTHERAPY DOSES**

VERFAHREN ZUR MESSUNG EINER RADIOTHERAPIE-DOSIS

PROCÉDÉ DE MESURE D'UNE DOSE DE RADIOTHÉRAPIE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **31.07.2017 IT 201700087851**

(43) Date of publication of application:
**10.06.2020 Bulletin 2020/24**

(73) Proprietor: **Istituto Nazionale Di Fisica Nucleare
(INFN)
00044 Frascati (RM) (IT)**

(72) Inventors:
• **CIRRONE, Giuseppe Antonio Pablo
95125 Catania (IT)**
• **ROMANO, Francesco
96100 Siracusa (IT)**
• **PETRINGA, Giada
95125 Catania (IT)**
• **AMATO, Antonino
95125 Catania (IT)**

(74) Representative: **Leone, Mario et al
Cantaluppi & Partners S.r.l.
VIA XX Settembre, 98g
00187 Roma (IT)**

(56) References cited:
**WO-A1-2011/113835**

EP 3 662 305 B1

**Description**

The present invention relates to a method for performing measurements in the field of radiotherapy, in particular of the dosage of ionizing radiations administered to a receiving subject, in particular a patient for the treatment, for example, of cancer pathologies or in diagnostic treatments such as computed tomography or *imaging* techniques using ionizing radiations. The invention further relates to an apparatus to perform said measurement.

Under ionizing radiations, radiations are meant provided with sufficient energy so as to ionize atoms or molecules of the tissues therewith they interact; different types thereof exist and they can be divided substantially into two groups: radiations which directly produce ions, i.e. comprising charged particles, such as α, β-, β+ particles, electrons, protons, which are directly ionizing; and the radiations which produce ions indirectly, and which comprise electromagnetic radiations such as γ or X rays and electrically neutral particles in general such as neutrons, which are called indirectly ionizing particles.

It is to be meant that the herein described method relates to both types of radiations the dosimetry thereof is fundamental for evaluating therapeutic effects or for controlling radioprotection protocols.

The current dosimetry methods substantially provide indirect measurements.

In the light of this, frequently one prefers to measure not so much the dose of radiations absorbed by the subject, but rather the radiations emitted by the radiotherapy apparatuses through specific outer detectors outside the subject, then without having a precise measurement of the effective impact of the radiations on the subject in the specific case.

International patent application No. WO 2011/113, 835 A1 describes a device and a method for controlling the radiotherapy quality.

The technical problem underlying the present invention consists in implementing a method allowing a measurement of the actually absorbed doses, performed not indirectly or by means of a sampling, but directly on the subject, and which then results to be more representative of the performed radiation.

The idea underlying the present invention is based upon the consideration that, whenever a subject, whether it is a patient or an equivalent tissue phantom, is subjected to a treatment with beams of ionizing radiations, on the treated tissues an unbalance of charge is induced or even produced, which is directly linked to the released dose.

In particular, a different effect can be seen depending upon the type of used radiation for the treatment:

- in case of treatments with directly ionizing, loaded particles inside the tissues an unbalance of net total charge takes place;
- in case of neutral particles or indirectly ionizing electromagnetic radiations a potential difference, due to medium ionization effects, is locally induced.

The end result of both these two processes is the generation of an electrical signal helpful to monitor the total dose absorbed during the therapy, and this allows to develop suitable electronic devices capable of measuring, with the required sensitivity, the voltage pulses and/or the net charge induced through the patient, i.e. through the subject under treatment, in cooperation with a specific analysis software capable both to calculate the real received dose and, under particular measurement conditions, to reconstruct the charge deposition site.

The above-mentioned technical problem then is solved by a method for measuring radiotherapy doses as defined in the appended claim 1, wherein a piece of information of substantially electrical type is exploited, which is inevitably produced by the tissues of the subject during the treatment. The quantities which are measured and required electronic devices can be different depending upon the type of used ionizing radiation:

- as far as the radiations with charged particles are concerned, such as electrons, protons and ions, the system allows to measure the voltage pulse deriving from the secondary ionization electrons set into motion and the accumulated net charge, which will be negative in case of electrons or positive in case of positive ions;
- in case of radiations with an electrically neutral radiation, i.e. either X and gamma photons or neutrons as well, the system allows to measure the voltage pulses deriving from having set in motion the secondary electrons inside the subject.

By extending the number of reading probes and by making the suitable changes to the acquisition system, it will be possible to perform even a position measurement therethrough the charge deposition point and, consequently, the spatial features (position and extension) of the dose deposition in the tissue could be reconstructed.

By way of example, the typical application of the present method could fall in the fields of in-vivo dosimetry in radiotherapy, for example performed with beams of photons, electrons, protons and ions and, generally, hadrons also generated by laser-material interaction, and that of radioprotection (of patients and workers), without neglecting environmental monitoring.

In particular, this methodology can be applied in the field of the in-vivo dosimetry as well as in the dose *on-line monitoring* in the field of the radiotherapy with ions: i.e. in the cases wherein the radiant beam does not come out of the patient and thus an immediate dose check is not possible.

It has to be further underlined that one of the major problems to be faced, with the purpose of performing an accurate measurement, is the reduction in the electronic noise. In fact, considering the low intensities of the measured signals which typically are lower than 1 nA, the electronic noise represents a not negligible disturbance element.

The low-frequency environmental electromagnetic pollution, for example produced by a simple household electric system, can indeed induce in human body a noise current at 50 Hz which can reach, typically, limit values of 200 nAp-p (peak-to-peak nanoAmpere).

In view of the above, with the purpose of obtaining a measurable signal, it is then necessary both to develop suitable reading electronic instruments and to implement a series of devices apt to minimize the electronic noise either generated by the subject through his/her possible motions or caused by the surrounding environment due to interferences which can be of electrostatic and/or electromagnetic nature.

Such devices involve both the total insulation of the subject with respect to the grounding and the removal of all outer electrostatic sources which can negatively affect the correct signal collection.

The invention further relates to an apparatus for performing the above-illustrated method, requiring the use of means for electrically insulating the subject, and at least a floating electrode and a system for amplifying the signal for each electrode, for detecting the voltage pulse produced during the treatment and deriving from the ionization secondary electrons set into motion and/or by the loaded net charge induced in the subject.

Said voltage pulse is converted by suitable means into a value of charge induced by the treatment in the subject, and a microprocessor provided with a storage and I/O devices for processing an analysis software then can reconstruct the charge deposit point and the calculation of the absolute and relative dose absorbed by the subject.

The proposed system could have at least two advantages among the current systems of in-vivo dosimetry used in hadron therapy. Such advantages lie in the system simplicity which would not require any applied voltage and which, differently from the other in-vivo dosimeters, would not absolutely disturb the radiation field since the sensor for the signal collection has not to be radiated.

Another advantage is linked to the potential possibility of being able to measure the absolute charge (and then the dose) absorbed by the patient in an absolute manner, hence without passing through a calibration, as it has to be done with current in-vivo dosimeters.

The proposed technique, if used by a take-over by several electrodes at the same time and by discriminating the different intensities of the signals, could allow a simple deduction of the charge deposition point, hence the possibility of having information linked to the real *range* of protons and ions.

Hereinafter, by pure way of not limiting example, an embodiment example of the present method and of possible variants comprised therein will be described, with reference to the enclosed drawings wherein:

- figure 1 shows a circuit diagram of a converter implemented to acquire signals in current of the beam incident on a subject by means of the positioning of one single electrode;
- figures 2A and 2B combined show a circuit diagram of two trans-impedance amplifiers implemented for deducing the incidence point of a beam on a patient by means of a current measurement obtained by using two electrodes;
- figure 3 shows a circuit diagram of a differential amplifier implemented for acquiring the signals in voltage of the incident beam on the patient by positioning three electrodes;
- figure 4 shows a graph of a signal obtained by means of the diagram of figure 1, acquired in-vitro, by using a water phantom, (lower curve) together with the beam current measured by means of a detector of SEM type (upper curve);
- figure 5 shows a graph illustrating a response curve in charge of the electrode depending upon the dose, obtained by means of the diagram of figure 1;
- figure 6 shows a graph of a signal obtained by means of the diagram of figure 1, acquired in-vivo (lower curve) together with the beam current measured by means a detector of SEM type (upper curve).

The herein described method, in its most general meaning, allows of evaluating both absolutely and relatively the dose supplied during a typical radiotherapy treatment, as well as of knowing the stop point of the loaded particles used in such treatment.

The main components of an apparatus for performing the method according to the invention can be schematized as follows:

a) a floating electrode, preferably of the disposal type, for example an electrode for electrocardiography;

b) a trans-impedance amplifier, also called current-voltage converter, used in case of direct measurement of the absorbed current;

c) a differential amplifier with high voltage gain, to be used in case of measurement of the potential differences produced by the incident radiation;

d) a system for acquiring the output signals generated by the previously mentioned amplifiers;

e) a microprocessor provided with a storage and I/O devices for processing an analysis software dedicated both to the reconstruction of the charge deposition point and to the calculation of the absolute and relative dose.

Depending upon the type of ionizing radiation used for the radiotherapy treatment, both the configuration of the acquisition system and the reconstruction software of the dose and the beam incidence point in the tissues have to be suitably selected.

Hereinafter, then, the different circuit configurations are described which it is possible to select to perform a correct in-vivo dosimetry during treatment.

Firstly, the case of radiotherapy treatments performed with loaded particles is faced, which require to determine the total dose released in the tissues and the beam incidence point.

Hereinafter, a converting circuit provided with one single electrode will be described.

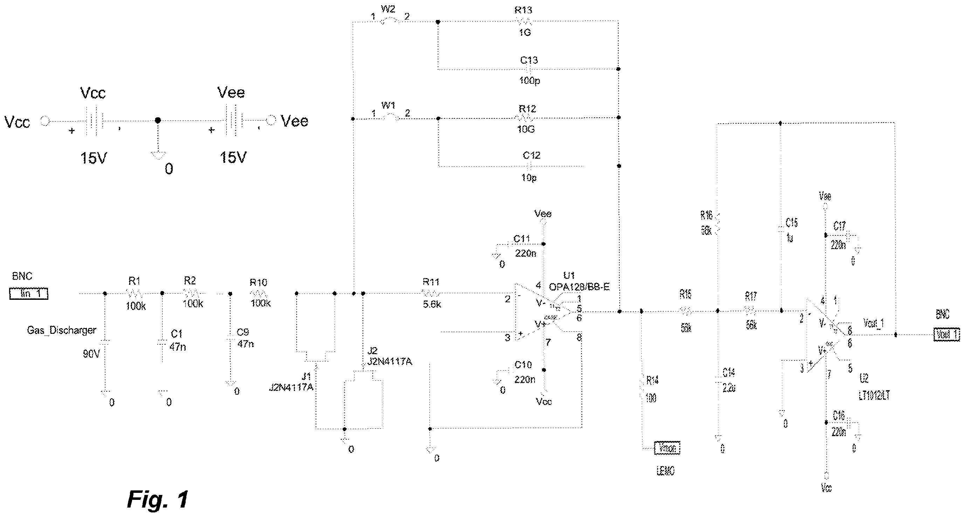

By referring to figure 1, by positioning a floating electrode of the disposal type on a patient subjected to a radiant treatment with beams of loaded particles (protons, ions, electrons), the invention allows to deduce the dose thereof absorbed by measuring the current absorbed by the patient.

The measurement of the current takes place by means of a trans-impedance amplifier having a low input impedance and suitably planned so that the current signal $I_{in}$, could be filtered and converted into an easily measurable voltage signal, by means of its transfer function: $V_{out} = R * I_{in}$.

Knowing that the currents induced by the beam of loaded particles are lower than the nano-Ampere, the amplifier is devised with a trans-impedance gain of $R = V_{out}/I_{in}$ having high ohmic value (typically $R \geq 10^9$ Ohm).

The circuit is shown in Figure 1 and it can be schematized in four fundamental portions:

The first portion of the circuit comprises a current-voltage converter (I-V) implemented by means of an operational amplifier OPA128 having electrometric degree and high performances, reactioned through a resistance of high ohmic value which establishes the trans-impedance gain $V_{out}/I_{in}$. The reaction resistance (R13 or R12) can be selected by means of a jumper and it can be selected among two possible values: 1 G$\Omega$ or 10 G$\Omega$. Parallely to the reaction resistance there is a condenser (respectively of 100 pF or 10 pF) the value thereof has been selected to meet the following conditions: to limit the higher cutting frequency of the converter to only 1.6 Hz; to guarantee a good stability margin of the whole feedback loop, thus by preventing the occurrence of self-oscillation thereof in the I-V converter; to guarantee a response to the step without over-elongations (*overshoot*). About the operating *range* of the input current $I_{in}$ it is required to consider that the converter has a linear behaviour in the output *range* $-10 \text{ V} \leq V_{out} \leq +10 \text{ V}$. It follows that by selecting 1-G$\Omega$ reaction resistance, the input operating *range* will be $-10 \text{ nA} \leq I_{in} \leq +10 \text{ nA}$. Besides, by selecting 10-G$\Omega$ reaction resistance, the operating *range* becomes $-1 \text{ nA} \leq I_{in} \leq +1 \text{ nA}$.

The second portion of the circuit comprises a passive low-pass filter, placed at the input of the circuit (see R1 to R10, C1 to C9) having a cutting frequency (-3 dB) of 0.9 Hz and an attenuation of -68 dB at the frequency of 50 Hz. This filter plays two fundamental roles:

- it protects OPA128 from the electrostatical discharges which can be induced both by the patient and by the medical staff assisting the patient during the pre- and post-treatment phases; and
- it attenuates by about a factor 2,500 the noise currents at 50 Hz which the patient inevitably produces due to the electromagnetic interferences (EMI) caused by the environmental electromagnetic pollution, the electrical energy distribution system thereof is the main cause.

The third portion of the circuit comprises a pair of Junction gate Field-Effect Transistors (JFET) for protecting OPA128 (J1, J2), which discharge to mass the input current $I_{in}$ in case the converter saturates. This condition takes place only and only if the current $I_{in}$ exceeds the measurement operating range of the converter I/V. On the contrary, the converter works within its operating range, the configuration of the whole circuit makes that the two JFETs insert a neglectable leakage current lower than 100 fA.

The fourth and last portion of the circuit comprises an inverting active low-pass filter of the second order and with two coincident poles (see configuration of U2), with the main task of cutting with a slope of -40 dB/decade the residual noise existing outside the I-V, starting from the frequency (-3 dB) of 1.2 Hz. The filter is selected of the inverting type to compensate the polarity inversion of the voltage existing at the converter output I-V. In this way the output voltage $V_{out}$ will be positive when the input current $I_{in}$ is positive too.

In the present example, the selection of limiting the band of the I-V converter at only 1 Hz arises from the need for finding the best possible compromise by keeping in mind both the need for amplifying the DC signal of interest, and the need for eliminating all noises which inevitably disturb the measurement; thereamong: the tribo-electric and piezoelectric noises caused by the mechanical motions of the target subject; the currents induced on the subject itself due to the effect of electromagnetic and/or electrostatic interferences.

In particular, the electromagnetic interference current at 50 Hz, of about 200 nAp-p, is subjected to an attenuation of:

- -68 dB by the first low-pass passive filter, placed at the input of the circuit, see R1 to R10, C1 to C9.

- -30 dB from the I-V Converter which comprises OPA128.

- -56 dB from the low-pass filter of the second order placed at the output, which comprises the integrated circuit LT1012.

Therefore, the overall attenuation of the noise current at 50 Hz will be equal to -154 dB, which means a factor of 1 / 50,000,000.

By considering the above-described I-V converter as a whole, in presence of a sufficiently steep input current step (≤1 msec), the rise time of the output voltage $V_{out}$ results to be about 0.6 seconds if measured between 10% and 90% of the variation $\Delta V_{out}$. whereas it results to be about 1.1 seconds if measured between 1% and 99% of the same variation $\Delta V_{out}$.

Moreover, by making a direct current analysis of the trans-impedance amplifier, it is known that the input impedance $R_{IN}$ results to be 1 MΩ (see R1+R2+ ... +R10). Given the above, knowing that the DC current which is to be measured tends to discharge to the ground by following the path of least resistance, it is necessary that the patient is well insulated ($R_{ISO}$ at least 1 GΩ of insulation) so that the current which has to be measured selects as path of least resistance the amplifier input.

At last, there is a second reason which imposes an insulation of the patient to the adequate ground ($R_{ISO}$), for example of at least 1 GΩ, which is dictated by the fact that the input impedance of the amplifier ($R_{IN}$) is connected to the virtual mass of OPA128, which has an offset voltage ($V_{OFF}$) which in the worst case could be +/- 500 μV. The presence of this small potential at the input of the trans-impedance amplifier induces the same amplifier to produce an error current $I_{ERR}$ which would false the measurement. This error current is equal to $I_{ERR}=V_{OFF}/(R_{IN}+R_{ISO})$.

The selection of using a specific electrode type, so as the selection of the materials which inevitably have to come in contact with the patient, is dictated by the need for reducing all effects which would compromise the measurement.

A floating electrode, for example, constituted by a metal disc made of AgCl immersed in a conductive gel, is an element which can be selected for this charge measurement, as it does not produce any significative disturb due to stresses of mechanical type.

As far as the treatment chair or couch is concerned, therewith the patient is inevitably in contact during the treatment, they can be optimized so that there are no signal dispersions. On this matter, they can be coated with an insulating material so as to hinder the grounding of the patient and consequently the flowing of the current towards the ground.

At the same time, all metal elements in direct contact with the beam of incident particles and placed near the patient have to be always placed at null potential, that is a ground connection has to be always guaranteed so that no induced charge effects are created which false the measurement.

As far as the software for reconstructing the absolute dose released to the patient is concerned, the calculation of the absolute dose can be performed starting from knowing the total charge absorbed by the patient and by the energy spectrum of the incident beam.

The absorbed dose in a beam of protons, in fact, is given by the relation:

$$D = \frac{\phi}{\varrho}\frac{dE}{dx} \quad (1)$$

wherein $\phi$ represents the fluence, that is the number of particles per square centimetre and it is measured in $cm^{-2}$, $\varrho$ is the density of the radiated material expressed in $Kg*cm^{-3}$ and at last, dE/dX is the value of the total *stopping power* to the energy of the incident beam expressed in in MeV/cm.

Once fixed the energy of the incident beam $E_0$, the system obtains the corresponding value of dE/dX starting from tabulated data (for example from tables ICRU49 in case of protons, ICRU73, in that of carbon). The value of fluence $\phi$ is calculated starting from the measured total charge and from the surface of the incident beam.

Typically, in a clinical treatment, the incident beam on the patient is polyenergetic. In this case, the dose can be calculated starting from the total charge read by the system by suitably correcting the previous definition of dose and by considering all energy components of the beam. In particular, the previous definition of dose has to be written by weighing suitably the fluence of each energy component of the spectrum depending upon the corresponding value of *stopping power*. According to what said, the following dose expression:

$$D = \sum_i \frac{\phi_i}{\varrho}\left(\frac{dE}{dx}\right)_i \quad (2)$$

is considered.

By using two or more electrodes, it is possible, under the same previously described operating conditions, having a piece of information about the beam incidence point during the treatment.

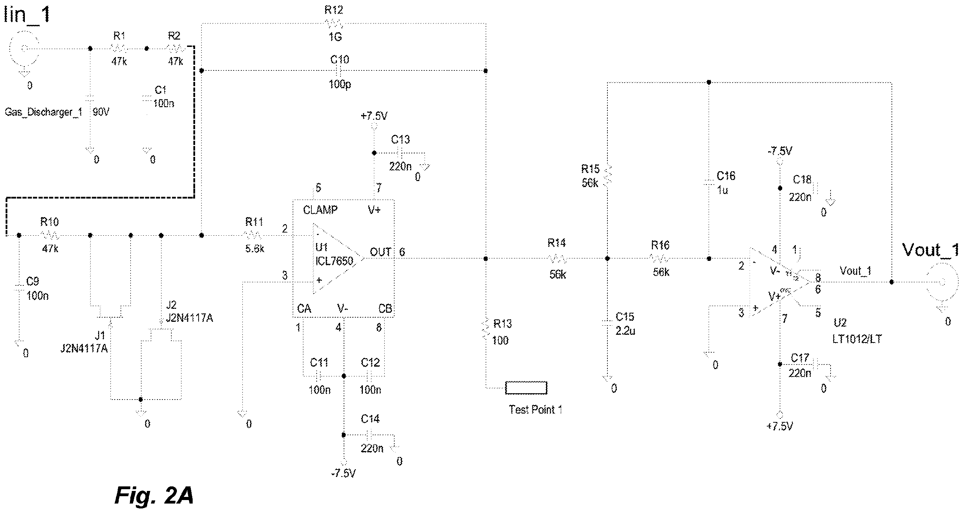

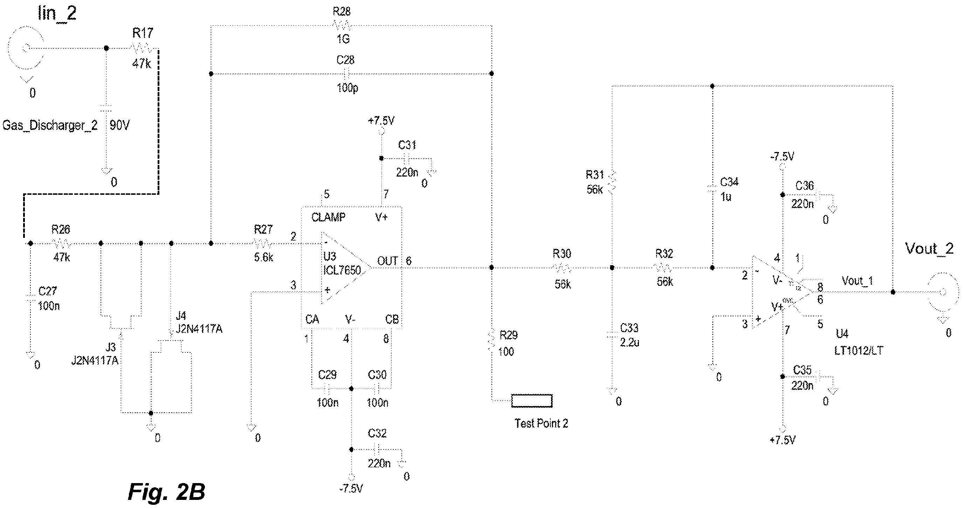

By referring to figure 2, this circuit is constituted by two twin trans-impedance amplifiers, assembled in the same card so as to share the same *ground,* that is the same reference potential within an error $\Delta V_{ERR}$ of only 1_μVdc. As it can be observed, the configuration adopted in each trans-impedance amplifier differs from that of Figure 1 since the input impedance is lower by a factor two with respect to the previous one and the conversion stage I-V is implemented by means of an operational amplifier model ICL7650 of *Chopper-Stabilized* type.

The sensitivity of the multi-electrode system, for detecting the charge deposition point, is based upon the evaluation of the different current intensity acquired at the same time by the two electrodes.

The ideal condition would be so that such diversity depended upon the different electrical resistance that each current meets upon crossing the tissues of the patient to reach the floating electrode. To say the truth, the two currents also meets the input impedance of the two trans-impedance amplifiers, which for this type of measurement represents a disturbing element to be corrected during the data analysis, therefore it is convenient that the input impedance of the two amplifiers is as small as possible, compatibly with the technological resources existing on the market.

Should the two trans-impedance amplifiers have been ideal, their inputs would have always the same electrical potential. In the real case, however, it is necessary to consider the potential difference $\Delta V_{OFF}$ between the two inputs $I_{IN\_1}$ and $I_{IN\_2}$. In particular, such potential difference is given by the following contributions $\Delta V_{OFF} = V_{OFF1} + V_{OFF2} + \Delta V_{ERR}$, wherein $V_{OFF1}$ and $V_{OFF2}$ are the input offset voltages of the two operational amplifiers U1 and U3, respectively, whereas $\Delta V_{ERR}$ is the difference in the *ground* electrical potential between pin 3 of U1 and pin 3 of U3, which as mentioned above will be no more than 1 pVdc.

Whenever the two electrodes are positioned on the patient, the presence of $\Delta V_{OFF}$ inevitably makes a small offset current ($I_{OFF}$) to flow both in the tissues of the patient himself/herself, and in each input of the two trans-impedance amplifiers.

The current intensity $I_{OFF}$ is not deterministic as it will depend even upon the electrical resistance which the same tissues of the patient have, however $I_{OFF}$ will be not higher than: $I_{OFF\_MAX} = \Delta V_{OFF}/2*R_{IN}$ wherein $R_{IN}$ = 470 kΩ corresponds to the input impedance of each amplifier under DC regime.

Therefore, if one wants to limit $I_{OFF\_MAX} \leq 15$ pA, it is necessary that the offset voltages $V_{OFF1}$ and $V_{OFF2}$, of U1 and U3 respectively, singularly are $\leq 5$ pVdc. This condition is met if and only if U1 and U3 are operational amplifiers of *Chopper-Stabilized* type. For this application the ICL7650 model is selected as, apart from satisfying the above condition, it is characterized by a polarization current $I_{BIAS\_typ} \leq 1.5$ pA.

The only drawback which has to be considered is that the operational amplifier ICL7650 works with a supply voltage of ± 7.5 Volt, therefore the linearity of its output voltage is guaranteed within the *range* of ± 5 Volt. Considering that U1 and U3 are polarized only by a 1-GΩ reaction resistance, it means that the operating *range* of the input current $I_{IN}$ of each amplifier will be limited to only -5 nA $\leq I_{IN} \leq$ +5 nA.

In the example with two electrodes the software for reconstructing the absolute dose release to the patient has a beam incidence point reconstruction algorithm which then is based upon the different current intensity measured by each electrode.

Whenever the beam of particles interacts with the tissues, a current will be produced which will branch inside the tissues themselves.

The time being equal, the charge measurement collected by one or more electrodes, in fact, can provide a useful piece of information about the distance between each electrode and the beam incidence point.

From the comparison of the signals produced by the electrodes and the knowledge of the position of the latter it is possible to detect the area wherein the beam of particles, by interacting with the tissues, has produced a current signal inside thereof.

An ad-hoc circuit was devised also for measuring the pulse due to the production of secondary electrons produced in the interaction of a ionizing radiation with the body.

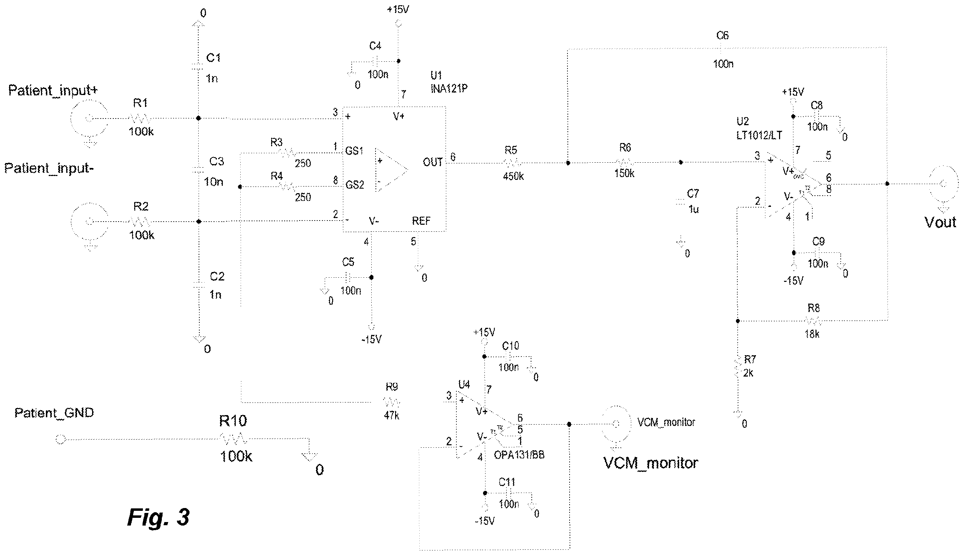

The scheme of the circuit shown in Figure 3 then allows to use the herein processed idea not only when a net charge is input into the patient, but even when a charge imbalance is generated locally after the passage of a radiation and the consequent generation of secondary electrons by ionization. Such imbalance is translated into a voltage pulse which could be measured and put in relation to the dose released to the patient by the incident radiation.

In particular, the circuit shown in Figure 3 consists in a differential amplifier with high input impedance ($10^{12}$ Ω), having an overall DC voltage gain equal to 1000 and a cutting frequency higher than 1 Hz.

The amplifier is equipped with an auxiliary output dedicated to monitor the voltage of common mode existing during the measurement. It is constituted by:

- a passive low-pass filter placed at the input of the circuit, which has the double purpose of filtering the component of the differential signal coming from the patient and, at the same time, to remove the performed RF disturbances of common mode which can induce problems of electromagnetic compatibility to the whole amplification system. The cutting frequency of common mode was fixed to 1.6 kHz.

- a differential amplifier for instrumentation (Instrumentation Amplifier) INA121P with field effect transistor (FET) input, configured for amplifying with a voltage gain equal to 100. Thanks to its features, this type of amplifier finds wide applications in the medical diagnosis field such as electrocardiography (ECG), electroencephalography (EEG) and electromyography (EMG).

- a not inverting active low-pass filter of the second order (see U2), which further amplifies the signal by a factor 10, by cutting the components of the signal having a frequency (-3 dB) equal to 1 Hz. In particular, the filter has the function of eliminating both the noises at 50 Hz linked to the environmental electromagnetic pollution and the noises linked to the biological nature of the patient (such as for example, the pulses of the nerve cells and the muscle contractions).

- an operational amplifier OPA131, configured as tracker, which allows to monitor the voltage of common mode (VCM) existing during the measurement.

Before concluding the description of the differential amplifier, it is necessary to specify that it was planned for working even during the measurement of the signal in current of the beam incident on the patient. In such case it will be necessary to replace the "Patient_GND" electrode existing in Figure 3, with the electrode indeed dedicated to the measurement of the current of the beam incident on the patient.

**Examples**

A series of preliminary tests was performed by using the circuit shown in Figure 1 and the previously described modes, by acquiring both in-vitro signals, by using a water phantom, and in-vivo signals, by acquiring the signals during a proton-therapy session on a patient.

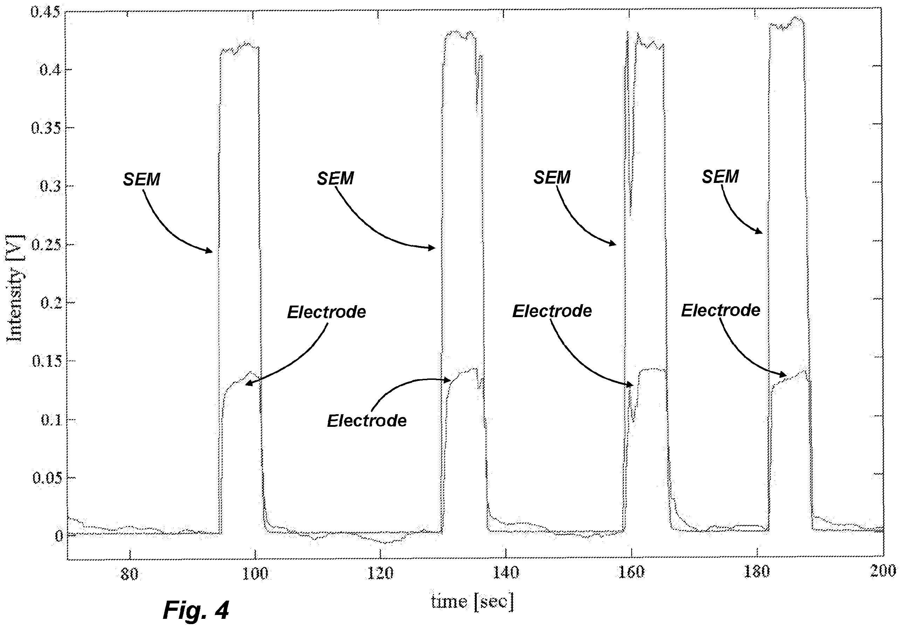

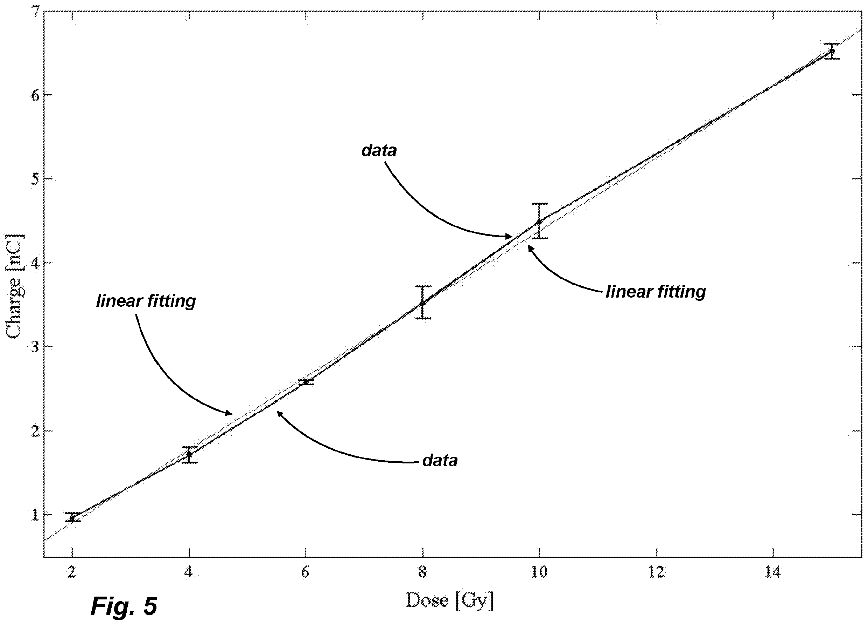

The results of such studies are shown in figures 4 and 5. Figure 4 shows the signal obtained by positioning the electrode inside the water phantom during a radiation with a clinical beam of 60-MeV protons with a dose equal to 4. The same graph shows both the signal of the electrode (lower curve) and the signal of a device of SEM type (Secondary Emission Monitoring, upper curve) used as online reference of the beam current supplied during the measurement.

Figure 5 shows the response curve of the loading electrode expressed as function of different values of absorbed dose. In this case, it can be noted that the electrode response follows an almost linear path in perfect agreement with the charge expected for fixed values of supplied dose.

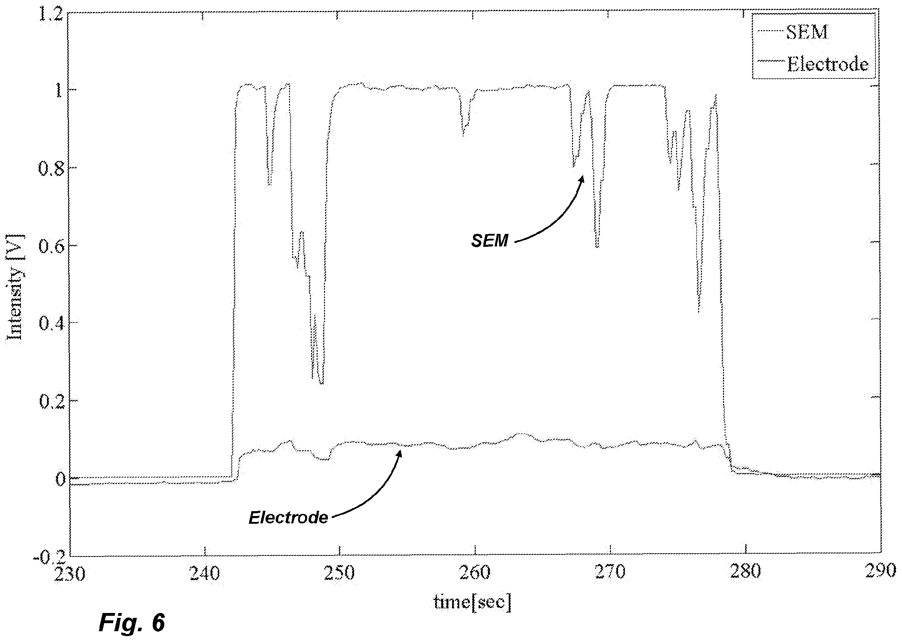

Figure 6 shows the system current response obtained during the measurement performed on patient, that is in vivo.

In this case, the patient, during the treatment, was subjected to a radiation by receiving a dose of 13.66 Gy. The value of supplied *dose rate* was equal to 25.62 Gy/min. As it is shown in Figure 6, the treatment starts at second 242 and it continues for 32 seconds. The current read by the system reaches the value of 0.9 V equal to 0.09 nA. The response of the same patient was monitored during the four clinical sessions provided by the protocols and a good reproducibility of the measurement was observed. The average of the total charges resulted to be equal to 3.82 nC with a standard deviation corresponding to 0.87.

To the above-described measuring method a person skilled in the art, with the purpose of satisfying additional and contingent needs, could introduce several additional modifications and variants, however all comprised within the protective scope of the present invention, as defined by the enclosed claims.

**Claims**

1. A method for measuring radiotherapy doses, in particular on a subject undergoing radiotherapy or other treatments with ionizing radiations, comprising the steps of:

   • electrically insulating the subject during the treatment;
   • applying at least two equal electrodes to the subject, each one connected to an amplifier with a system for acquiring the signal outgoing from the amplifier;
   • detecting, by means of said at least two electrodes, a voltage pulse produced during the treatment, said voltage pulse deriving from the ionization secondary electrons set into motion and/or from the net charge induced in the subject;
   • converting said voltage pulse into a value of charge induced by the treatment in the subject; and
   • determining the dose of ionizing radiations received by the subject by means of a processing system which uses the above-mentioned value of the induced charge, an energy spectrum of the incident beam of ionizing radiations and a contact surface of said incident beam on the subject;
   • evaluating the different intensity of current acquired at the same time by said at least two electrodes and of the extent of the induced charge detected by each electrode, and determining the position of a region of the subject wherein the incident beam, by interacting with the subject's tissues, has produced a current signal inside thereof, by comparing the signals produced by the electrodes and the knowledge of their position.

2. The method according to claim 1, wherein the ionizing radiations are of the type selected from the group comprising protons, electrons, ions, neutrons, X radiations and gamma radiations.

3. The method according to one of the previous claims, wherein:

   a) at least a floating electrode;
   b) a system for amplifying the signal for each electrode
   c) a system for acquiring the output signals generated by said amplifiers;
   d) a microprocessor provided with a storage and I/O devices for processing an analysis software dedicated both to the reconstruction of the charge deposition point and to the calculation of the absolute and relative dose

**4.** The method according to claim 3, wherein the amplifying system comprises:

a) a trans-impedance amplifier for the direct measurement of the absorbed current; and/or
b) a high voltage gain differential amplifier, for the measurement of the potential differences produced by the incident radiation.

**5.** The method according to claim 4, wherein the trans-impedance amplifier comprises:

• a current-voltage converter (I-V) having an output linear behaviour;
• a passive low-pass filter having a predetermined cutting frequency and attenuation for protecting the converter and for reducing the environmental noise;
• a pair of protecting junction field effect transistors (JFET) which discharge to mass the input current in case the converter saturates; and
• an inverting active low-pass filter of the second order and with two coincident poles to compensate the polarity inversion of the voltage existing at the converter output.

**6.** The method according to claim 5, wherein the converter comprises a condenser the value thereof limits the upper cutting frequency of the converter to a predetermined value.

**7.** The method according to claim 4, wherein the high voltage gain differential amplifier comprises:

• a passive low-pass filter placed at the input, to filter the component of the differential signal coming from the subject and, at the same time, to remove the RF disturbances with a predetermined cutting frequency;
• a differential amplifier for differential instruments with field effect transistor (FET) input;
• a not inverting active low-pass filter of the second order to remove the environmental noises; and
• an operational amplifier, configured as tracker, which allows to monitor the voltage of common mode (VCM) existing during the measurement, with a corresponding auxiliary output.

**8.** An apparatus for measuring radiotherapy doses, in particular on a subject undergoing radiotherapy or other treatments with ionizing radiations, comprising:

• means for electrically insulating the subject;
• at least two equal floating electrodes applied to the subject and a system for amplifying the signal for each electrode for detecting the voltage pulse produced during the treatment, said voltage pulse deriving from the ionization secondary electrons set into motion and/or by the loaded net charge induced in the subject;
• means for converting said voltage pulse into a value of charge induced by the treatment in the subject; and
• a microprocessor provided with a storage and I/O devices and configured to process an analysis software dedicated both to the reconstruction of the charge deposition point and to the calculation of the absolute and relative dose, wherein the above analysis software is set to evaluate a comparison of the different intensity of current acquired at the same time by said at least two electrodes and of the extent of the induced charge detected by each electrode, thereby, considering the electrode position, to further determine a region of the subject, wherein the incident beam, by interacting with the subject's tissues, has produced a current signal inside thereof.

**9.** The apparatus according to claim 8, wherein the amplifying system comprises:

• a trans-impedance amplifier for the direct measurement of the absorbed current; and/or
• a high voltage gain differential amplifier, for the measurement of the potential differences produced by the incident radiation.

**10.** The apparatus according to claim 9, wherein the trans-impedance amplifier comprises:

• a current-voltage converter (I-V) having an output linear behaviour;
• a passive low-pass filter having a predetermined cutting frequency and attenuation for protecting the converter and for reducing the environmental noise;
• a pair of protecting junction field effect transistors (JFET) which discharge to mass the input current in case the converter saturates; and
• an inverting active low-pass filter of the second order and with two coincident poles to compensate the polarity inversion of the voltage existing at the converter output.

**11.** The apparatus according to claim 10, wherein the converter comprises a condenser the value thereof limits the upper cutting frequency of the converter to a predetermined value.

**12.** The apparatus to according to claim 9, wherein the high voltage gain differential amplifier comprises:

• a passive low-pass filter placed at the input, to filter the component of the differential signal coming from the subject and, at the same time, to remove the RF disturbances with a predetermined cutting frequency;
• a differential amplifier for differential instruments with field effect transistor (FET) input;
• a not inverting active low-pass filter of the second order to remove the environmental noises; and
• an operational amplifier, configured as tracker, which allows to monitor the voltage of common mode (VCM) existing during the measurement, with a corresponding auxiliary output.

**Patentansprüche**

1. Verfahren zum Messen von Strahlentherapiedosen, insbesondere bei einem Subjekt, das sich einer Strahlentherapie oder anderen Behandlungen mit ionisierender Strahlung unterzieht, aufweisend die Schritte:

   • das Subjekt während der Behandlung elektrisch zu isolieren;
   • Anbringen mindestens zweier gleicher Elektroden an dem Subjekt, wobei jede mit einem Verstärker mit einem System zum Erfassen des vom Verstärker ausgehenden Signals verbunden ist;
   • Erfassen, mittels der mindestens beiden Elektroden, eines Spannungspulses, der während der Behandlung erzeugt wird, wobei der Spannungspuls von der Ionisation von in Bewegung versetzten Sekundärelektronen und/oder von der in dem Subjekt induzierten Nettoladung abgeleitet wird;
   • Umwandeln des Spannungspulses in einen Ladungswert, der durch die Behandlung in dem Subjekt induziert wird; und
   • Bestimmen der von dem Subjekt empfangenen Dosis ionisierender Strahlung mittels eines Verarbeitungssystems, das den oben erwähnten Wert der induzierten Ladung, ein Energiespektrum des einfallenden Strahls ionisierender Strahlung und eine Kontaktfläche des einfallenden Strahls auf dem Subjekt verwendet;
   • Auswerten der unterschiedlichen Stromstärke, die gleichzeitig von den mindestens zwei Elektroden erlangt wird, und des Ausmaßes der induzierten Ladung, die von jeder Elektrode detektiert wird, und Bestimmen der Position eines Bereichs des Subjekts, wobei der einfallende Strahl, durch Wechselwirkung mit dem Gewebe des Subjekts, darin ein Stromsignal erzeugt hat, durch Vergleich der von den Elektroden erzeugten Signale und der Kenntnis ihrer Position.

2. Verfahren nach Anspruch 1, wobei die ionisierenden Strahlungen von der Art sind, die aus der Gruppe ausgewählt ist, aufweisend Protonen, Elektronen, Ionen, Neutronen, Röntgenstrahlung und Gammastrahlung.

3. Verfahren nach einem der vorhergehenden Ansprüche, wobei:

   a) mindestens eine schwebende Elektrode;
   b) ein System zum Verstärken des Signals für jede Elektrode;
   c) ein System zum Erfassen der von den Verstärkern erzeugten Ausgangssignale;
   d) ein Mikroprozessor, der mit einem Speicher und E/A-Vorrichtungen zum Verarbeiten einer Analysesoftware bereitgestellt ist, die sowohl der Rekonstruktion des Ladungsablagerungspunkts als auch der Berechnung der absoluten und relativen Dosis gewidmet ist,

   verwendet werden.

4. Verfahren nach Anspruch 3, wobei das Verstärkungssystem aufweist:

   a) einen Transimpedanzverstärker zur direkten Messung des absorbierten Stroms; und/oder
   b) einen Differenzverstärker mit hoher Spannungsverstärkung zur Messung der durch die einfallende Strahlung erzeugten Potentialdifferenzen.

5. Verfahren nach Anspruch 4, wobei der Transimpedanzverstärker aufweist:

   • einen Strom-Spannungs-Wandler (I-V) mit linearem Ausgangsverhalten;
   • ein passives Tiefpassfilter mit einer vorbestimmten Grenzfrequenz und Dämpfung zum Schutz des Wandlers und zum Reduzieren des Umgebungsrauschens;
   • ein Paar Schutzsperrschicht-Feldeffekttransistoren (JFET), die sich entladen, um den Eingangsstrom auf das höchste Maß zu bringen, falls der Wandler in die Sättigung geht; und
   • ein invertierendes aktives Tiefpassfilter zweiter Ordnung mit zwei übereinstimmenden Polen zur Kompensation der Polaritätsumkehrung der am Wandlerausgang anliegenden Spannung.

6. Verfahren nach Anspruch 5, wobei der Wandler einen Kondensator aufweist, dessen Wert die obere Grenzfrequenz des Wandlers auf einen vorbestimmten Wert begrenzt.

7. Verfahren nach Anspruch 4, wobei der Differenzverstärker mit hoher Spannungsverstärkung aufweist:

• ein passives Tiefpassfilter, das am Eingang angeordnet ist, um die vom Subjekt kommende Komponente des Differenzsignals zu filtern und, gleichzeitig, um die RF-Störungen mit einer vorbestimmten Grenzfrequenz zu entfernen;
• einen Differenzverstärker für Differenzinstrumente mit Feldeffekttransistor (FET)-Eingang;
• ein nicht invertierendes aktives Tiefpassfilter zweiter Ordnung zum Entfernen von Umgebungsgeräuschen; und
• einen als Tracker konfigurierten Operationsverstärker, der es ermöglicht, die während der Messung vorhandene Gleichtaktspannung (VCM) mit einem korrespondierenden Hilfsausgang zu überwachen.

8. Vorrichtung zum Messen von Strahlentherapiedosen, insbesondere bei einem Subjekt, das sich einer Strahlentherapie oder anderen Behandlungen mit ionisierender Strahlung unterzieht, aufweisend:

• Mittel zum elektrischen Isolieren des Subjekts;
• mindestens zwei gleiche schwebende Elektroden, die dem Subjekt zugeordnet sind, und ein System zum Verstärken des Signals für jede Elektrode zum Erfassen des Spannungspulses, der während der Behandlung erzeugt wird, wobei der Spannungspuls von der Ionisation von in Bewegung versetzten Sekundärelektronen und/oder von der in dem Subjekt induzierten Nettoladung abgeleitet wird;
• Mittel zum Umwandeln des Spannungspulses in einen Ladungswert, der durch die Behandlung in dem Subjekt induziert wird; und
• einen Mikroprozessor, der mit einem Speicher und E/A-Vorrichtungen zum Verarbeiten bereitgestellt ist und der konfiguriert ist, um eine Analysesoftware zu verarbeiten, die sowohl der Rekonstruktion des Ladungsablagerungspunkts als auch der Berechnung der absoluten und relativen Dosis gewidmet ist, wobei die obige Analysesoftware eingestellt ist zum Auswerten eines Vergleichs der verschiedenen Stromstärken, die gleichzeitig von den mindestens zwei Elektroden erlangt wird, und des Ausmaßes der induzierten Ladung, die von jeder Elektrode detektiert wird, wobei dabei die Position der Elektrode berücksichtigt wird, um weiterhin einen Bereich des Subjekts zu bestimmen, wo der Einfallstahl, durch Interaktion mit dem Gewebe des Subjekts, darin ein Stromsignal erzeugt hat.

9. Vorrichtung nach Anspruch 8, wobei das Verstärkungssystem aufweist:

• einen Transimpedanzverstärker zur direkten Messung des absorbierten Stroms; und/oder
• einen Differenzverstärker mit hoher Spannungsverstärkung zur Messung der durch die einfallende Strahlung erzeugten Potentialdifferenzen.

10. Vorrichtung nach Anspruch 9, wobei der Transimpedanzverstärker aufweist:

• einen Strom-Spannungs-Wandler (I-V) mit linearem Ausgangsverhalten;
• ein passives Tiefpassfilter mit einer vorbestimmten Grenzfrequenz und Dämpfung zum Schutz des Wandlers und zum Reduzieren des Umgebungsrauschens;
• ein Paar Schutzsperrschicht-Feldeffekttransistoren (JFET), die sich entladen, um den Eingangsstrom auf das höchste Maß zu bringen, falls der Wandler in die Sättigung geht; und
• ein invertierendes aktives Tiefpassfilter zweiter Ordnung mit zwei übereinstimmenden Polen zur Kompensation der Polaritätsumkehrung der am Wandlerausgang anliegenden Spannung.

11. Vorrichtung nach Anspruch 10, wobei der Wandler einen Kondensator aufweist, dessen Wert die obere Grenzfrequenz des Wandlers auf einen vorbestimmten Wert begrenzt.

12. Vorrichtung nach Anspruch 10, wobei der Differenzverstärker mit hoher Spannungsverstärkung aufweist:

• ein passives Tiefpassfilter, das am Eingang angeordnet ist, um die vom Subjekt kommende Komponente des Differenzsignals zu filtern und, gleichzeitig, um die RF-Störungen mit einer vorbestimmten Grenzfrequenz zu entfernen;
• einen Differenzverstärker für Differenzinstrumente mit Feldeffekttransistor (FET)-Eingang;
• ein nicht invertierendes aktives Tiefpassfilter zweiter Ordnung zum Entfernen von Umgebungsgeräuschen; und
• einen als Tracker konfigurierten Operationsverstärker, der es ermöglicht, die während der Messung vorhandene Gleichtaktspannung (VCM) mit einem korrespondierenden Hilfsausgang zu überwachen.

**Revendications**

1. Procédé de mesure de doses de radiothérapie, en particulier sur un sujet subissant une radiothérapie ou d'autres traitements avec des rayonnements ionisants, comprenant les étapes consistant à :

• isoler électriquement le sujet pendant le traitement ;
• appliquer au moins deux électrodes égales sur le sujet, chacune étant connectée à un amplificateur avec un système d'acquisition du signal sortant de l'amplificateur ;

• détecter, au moyen desdites au moins deux électrodes, une impulsion de tension produite pendant le traitement, ladite impulsion de tension dérivant des électrons secondaires d'ionisation mis en mouvement et/ou de la charge nette induite dans le sujet ;

• convertir ladite impulsion de tension en une valeur de charge induite par le traitement chez le sujet ; et

• déterminer la dose de rayonnements ionisants reçue par le sujet au moyen d'un système de traitement qui utilise la valeur susmentionnée de la charge induite, un spectre d'énergie du faisceau incident de rayonnements ionisants et une surface de contact dudit faisceau incident sur le sujet ;

• évaluer l'intensité différente de courant acquise en même temps par lesdites au moins deux électrodes et de l'importance de la charge induite détectée par chaque électrode, et

déterminer la position d'une région du sujet dans laquelle le faisceau incident, en interagissant avec les tissus du sujet, a produit un signal de courant à l'intérieur de ceux-ci, par la comparaison des signaux produits par les électrodes et la connaissance de leur position.

2. Procédé selon la revendication 1, dans lequel les rayonnements ionisants sont du type sélectionné dans le groupe comprenant les protons, les électrons, les ions, les neutrons, les rayonnements X et les rayonnements gamma.

3. Procédé selon l'une des revendications précédentes, dans lequel :

a) au moins une électrode flottante ;
b) un système d'amplification du signal pour chaque électrode
c) un système d'acquisition des signaux de sortie générés par lesdits amplificateurs ;
d) un microprocesseur doté d'un stockage et de dispositifs E/S pour traiter un logiciel d'analyse dédié à la fois à la reconstruction du point de dépôt de charge et au calcul de la dose absolue et relative

sont utilisés.

4. Procédé selon la revendication 3, dans lequel le système d'amplification comprend :

a) un amplificateur de transimpédance pour la mesure directe du courant absorbé ; et/ou

b) un amplificateur différentiel à gain de tension élevée, pour la mesure des différences de potentiel produites par le rayonnement incident.

5. Procédé selon la revendication 4, dans lequel l'amplificateur de transimpédance comprend :

• un convertisseur courant-tension (I-V) ayant un comportement linéaire en sortie ;
• un filtre passe-bas passif ayant une fréquence de coupure et une atténuation prédéterminées pour protéger le convertisseur et pour réduire le bruit ambiant ;
• une paire de transistors à effet de champ à jonction (JFET) de protection qui déchargent vers la masse le courant d'entrée en cas de saturation du convertisseur ; et
• un filtre passe-bas actif inverseur du deuxième ordre et à deux pôles coïncidents pour compenser l'inversion de polarité de la tension existant à la sortie du convertisseur.

6. Procédé selon la revendication 5, dans lequel le convertisseur comprend un condensateur dont la valeur limite la fréquence de coupure supérieure du convertisseur à une valeur prédéterminée.

7. Procédé selon la revendication 4, dans lequel l'amplificateur différentiel à gain de tension élevée comprend :

• un filtre passe-bas passif placé à l'entrée, pour filtrer la composante du signal différentiel provenant du sujet et, en même temps, pour supprimer les perturbations RF avec une fréquence de coupure prédéterminée ;
• un amplificateur différentiel pour instruments différentiels avec une entrée à transistor à effet de champ (FET) ;
• un filtre passe-bas actif non-inverseur du deuxième ordre pour supprimer les bruits ambiants ; et
• un amplificateur opérationnel, configuré en suiveur, qui permet de surveiller la tension de mode commun (VCM) existant pendant la mesure, avec une sortie auxiliaire correspondante.

8. Appareil de mesure de doses de radiothérapie, en particulier sur un sujet subissant une radiothérapie ou d'autres traitements avec des rayonnements ionisants, comprenant :

• des moyens pour isoler électriquement le sujet ;
• au moins deux électrodes flottantes égales appliquées sur le sujet et un système d'amplification du signal pour chaque électrode pour détecter l'impulsion de tension produite pendant le traitement, ladite impulsion de tension dérivant des électrons secondaires d'ionisation mis en mouvement et/ou de la charge nette chargée induite dans le sujet ;

• des moyens pour convertir ladite impulsion de tension en une valeur de charge induite par le traitement chez le sujet ; et

• un microprocesseur doté d'un stockage et de dispositifs E/S et configuré pour exécuter un logiciel d'analyse dédié à la fois à la reconstruction du point de dépôt des charges et au calcul de la dose absolue et relative,

dans lequel le logiciel d'analyse ci-dessus est paramétré pour évaluer une comparaison des différences d'intensité de courant acquises en même temps par lesdites au moins deux électrodes et de l'importance de la charge induite détectée par chaque électrode, ainsi, en tenant compte de la position de l'électrode, pour déterminer en outre une région du sujet dans laquelle le faisceau incident, en interagissant avec les tissus du sujet, a produit un signal de courant à l'intérieur de ceux-ci.

9. Appareil selon la revendication 8, dans lequel le système d'amplification comprend :

• un amplificateur de transimpédance pour la mesure directe du courant absorbé ; et/ou

• un amplificateur différentiel à gain de tension élevée, pour la mesure des différences de potentiel produites par le rayonnement incident.

10. Appareil selon la revendication 9, dans lequel l'amplificateur de transimpédance comprend :

• un convertisseur courant-tension (I-V) ayant un comportement linéaire en sortie ;

• un filtre passe-bas passif ayant une fréquence de coupure et une atténuation prédéterminées pour protéger le convertisseur et pour réduire le bruit ambiant ;

• une paire de transistors à effet de champ à jonction (JFET) de protection qui déchargent vers la masse le courant d'entrée en cas de saturation du convertisseur ; et

• un filtre passe-bas actif inverseur du deuxième ordre et à deux pôles coïncidents pour compenser l'inversion de polarité de la tension existant à la sortie du convertisseur.

11. Appareil selon la revendication 10, dans lequel le convertisseur comprend un condensateur dont la valeur limite la fréquence de coupure supérieure du convertisseur à une valeur prédéterminée.

12. Appareil selon la revendication 9, dans lequel l'amplificateur différentiel à gain de tension élevée comprend :

• un filtre passe-bas passif placé à l'entrée, pour filtrer la composante du signal différentiel provenant du sujet et, en même temps, pour supprimer les perturbations RF avec une fréquence de coupure prédéterminée ;

• un amplificateur différentiel pour instruments différentiels avec une entrée à transistor à effet de champ (FET) ;

• un filtre passe-bas actif non-inverseur du deuxième ordre pour supprimer les bruits ambiants ; et

• un amplificateur opérationnel, configuré en suiveur, qui permet de surveiller la tension de mode commun (VCM) existant pendant la mesure, avec une sortie auxiliaire correspondante.

*Fig. 1*

*Fig. 2A*

Fig. 2B

EP 3 662 305 B1

*Fig. 3*

*Fig. 4*

*Fig. 5*

**Fig. 6**

EP 3 662 305 B1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2011113 A
- WO 835 A1